# EUROPEAN PATENT SPECIFICATION

(11) **EP 1 825 834 B1**
(45) Date of publication and mention of the grant of the patent: **21.12.2011**
(21) Application number: 07250622.3
(22) Date of filing: 15.02.2007
(51) Int. Cl.: A61F 2/34

(54) **Apparatus for aligning a taper lock connection**
Vorrichtung zur Zuweisung einer Kegelbefestigungsverbindung
Appareil d'alignement d'une connexion de verrouillage conique

(30) Priority: 28.02.2006 US 365895
(43) Date of publication of application: 29.08.2007
(73) Proprietor: Biomet Manufacturing Corp., Warsaw, IN 46581-0587 (US)
(72) Inventor: Slone, W. Jason, Silver Lake Indiana 46982 (US)
(74) Representative: Giles, Ashley Simon

(56) References cited:
- CH-A5- 677 072
- FR-A1- 2 595 562
- FR-A1- 2 855 743
- US-A- 5 147 407

## Description

### FIELD

The present teachings relate generally to implants, and particularly to an apparatus for aligning a taper lock connection on an acetabular implant.

### BACKGROUND

Many portions of the human anatomy naturally articulate relative to one another. Generally, the articulation between the portions of the anatomy is substantially smooth and without abrasion. This articulation is allowed by the presence of natural tissues, such as cartilage and strong bone.

Over time, however, due to injury, stress, degenerative health issues and various other issues, articulation of the various portions of the anatomy can become rough or impractical. For example, injury can cause the cartilage or the boney structure to become weak, damaged, or non-existent. Therefore, the articulation of the anatomical portions is no longer possible for the individual.

At such times, it can be desirable to replace the anatomical portions with a prosthetic portion such that normal or easy articulation can be reproduced. A femur naturally articulates within an acetabulum surface or cavity in a pelvis. After injury or other degenerative processes, the acetabulum can become rough or damaged. Therefore, it can be desirable to replace the acetabulum with a prosthesis.

Various prostheses exist for the acetabulum. Generally, a prosthesis includes a two-piece configuration with a bearing liner residing in an outer shell or acetabular cup. If the bearing liner is not properly aligned with the acetabular cup, the bearing liner can fracture or inadvertently be placed in the wrong orientation during assembly, resulting in increased cost and operation time. In addition, many of the prostheses available for the acetabulum require external fasteners or fasteners disposed in the bearing liner to secure the bearing liner to the acetabular cup. The use of external fasteners or fasteners within the housing can increase assembly time and cost. Accordingly, it can be desirable to provide an acetabulum prosthesis with an integral alignment and locking mechanism.
FR 2595562 discloses a prosthesis comprising cooperating cupules, corresponding surfaces of which comprise a cylindrical portion, a truncated conical portion and a flat portion. CH 677072 discloses a prosthesis comprising a conical metal shell that receives a conical core having a curved recess. US 5147407 discloses a prosthesis comprising an insert that is received within an inner shell. The insert and inner shell comprise cooperating conical surfaces. FR 2855743 discloses a prosthesis comprising a shell and a lining, corresponding surfaces of which comprise a cylindrical portion and a conical portion. The lining is formed within the shell using a thermo-compression method.

### SUMMARY

According to the present invention, there is provided a prosthesis for replacing a portion of the anatomy as disclosed in claim 1. Optional advantageous features of the present invention are disclosed in the dependent claims.

According to various embodiments of the invention, the prosthesis is an acetabular prosthesis including an acetabular shell defining the first alignment portion and the first taper, and a liner defining the second alignment portion and the second taper.

A method for replacing a portion of the anatomy is disclosed in the present specification but is not claimed. The method includes preparing a surface of the anatomy. The method can further include providing a first body extending between a first region and a second region defining a first alignment portion near the first region and a first taper near the second region. The method can also include providing a second body extending between a third region and a fourth region defining a second alignment portion near the third region and a second taper near the fourth region. The method can also comprise aligning the first alignment portion with the second alignment portion and locking the second body within the first body by enabling the first taper to interact with the second taper.

Further areas of applicability of the present teachings will become apparent from the detailed description provided hereinafter. It should be understood that the detailed description and various examples, while indicating various embodiments, are intended for purposes of illustration only and are not intended to limit the scope of the claims.

### BRIEF DESCRIPTION OF THE DRAWINGS

The present teachings will become more fully understood from the detailed description and the accompanying drawings, wherein:

Fig. 1 is a perspective view of a prosthesis, such as an acetabular cup, employing a taper lock connection and taper lock mechanism according to various teachings;

Fig. 2A is a first perspective exploded view of the acetabular - cup of Fig. 1;

Fig. 2B is a second perspective exploded view of the acetabular cup of Fig. 1;

Fig. 3 is a cross-sectional view of the acetabular cup and taper lock mechanism of Fig. 1, taken along line 3-3, illustrating the effect of misaligning the taper lock mechanism;

Fig. 4 is a cross-sectional view of the acetabular cup and taper lock mechanism of Fig. 1, taken along line 3-3, illustrating the engagement of the taper lock mechanism;

Fig. 5 is an environmental view of a procedure employed to prepare a selected portion of the anatomy for receipt of the acetabular cup and taper lock mechanism according to various teachings;

Fig. 6 is an environmental view of the acetabular cup and taper lock mechanism prior to engagement with the portion of the anatomy;

Fig. 7 is an environmental view of the acetabular cup and taper lock mechanism fully engaged with the portion of the anatomy;

Fig. 8 is an acetabular cup employing a fixation method according to various teachings;

Fig. 9 is an environmental view of a procedure employed to prepare a selected portion of the anatomy for receipt of the acetabular cup according to various teachings;

Fig. 10 is an environmental view of a procedure employed to engage the acetabular cup according to various teachings;

Fig. 11 is an environmental view of the acetabular cup engaged with the anatomy; and

Fig. 12 is an environmental view of a femoral head being assembled into the acetabular cup according to the various teachings.

### DETAILED DESCRIPTION OF VARIOUS EMBODIMENTS

The following description of various embodiments is merely exemplary in nature and is in no way intended to limit the teachings, its application, or uses. Although the following description is related generally to a prosthesis that can be positioned in a prepared portion of the anatomy, such as in an acetabulum in the pelvis, it will be understood that the prosthesis, as described and claimed herein, can be used with any appropriate surgical procedure. Therefore, it will be understood that the following discussions are not intended to limit the scope of the appended claims.

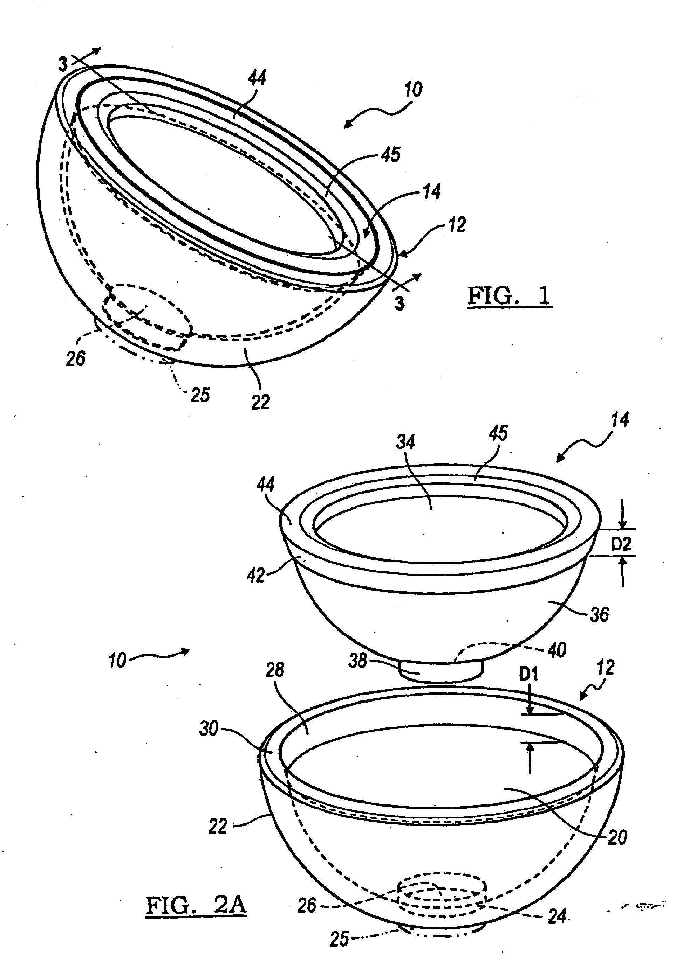

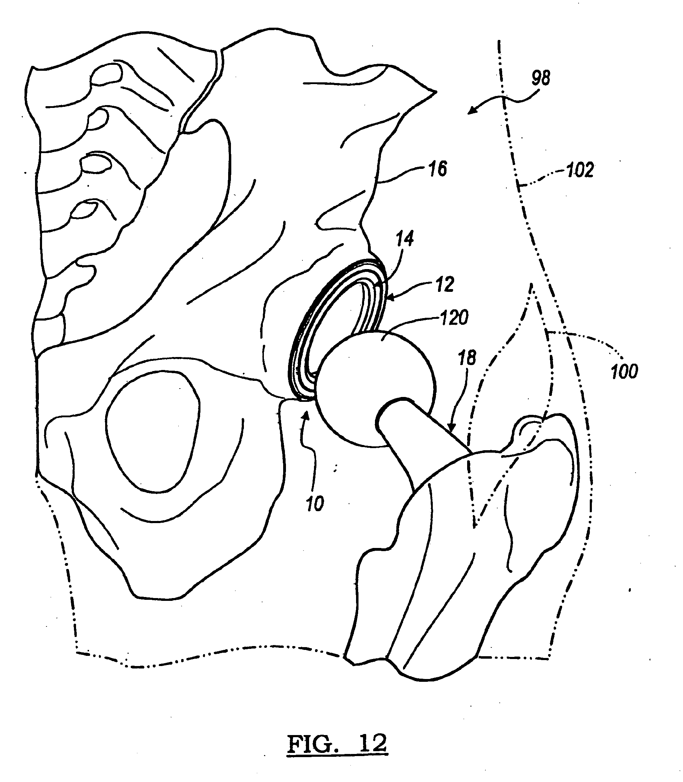

As will be discussed in more detail herein, an acetabular prosthesis assembly 10 is taught. With reference to Fig. 1, the acetabular prosthesis assembly 10 can include an acetabular shell 12 and a bearing liner 14. The acetabular prosthesis assembly 10 can be secured to a portion of the anatomy, such as a pelvis 16, for receipt of a second portion of the anatomy, such as a natural femur or a femoral prosthesis 18, which can include either a natural femoral head or femoral head prosthesis 120, as best shown in Fig. 12.

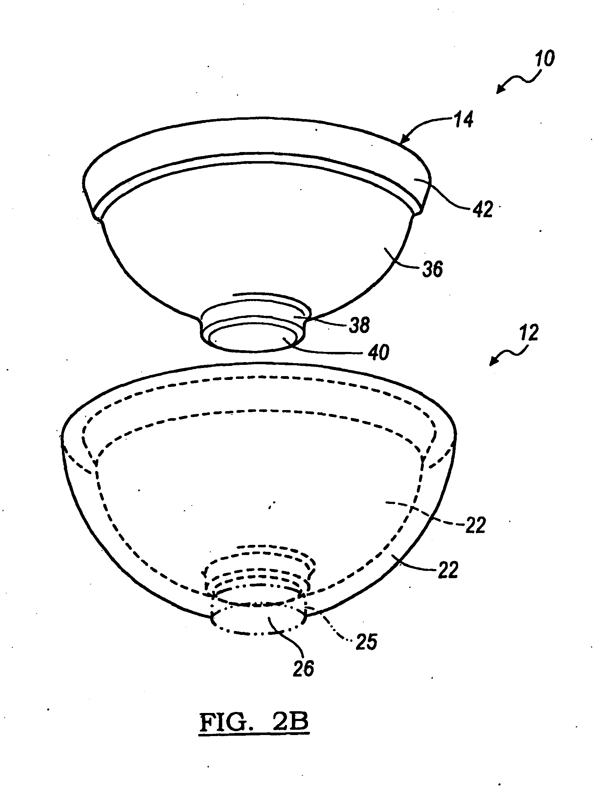

With additional reference to Figs. 2A, 2B, 3 and 4, the acetabular shell 12 includes a first or interior surface 20 and a second or exterior bone engaging surface 22. The acetabular shell 12 is generally a hemispherical. The acetabular shell 12 can be composed of a bio-compatible metallic material, such as titanium, titanium alloy, stainless steel, cobalt-chromium-molybedenum alloy, but any other bio-compatible material, such as a polymeric material, could be employed. The interior surface 20 is generally concave and have a selected texture, such as smooth. The interior surface 20 includes an alignment portion 21, and the alignment portion 21 can include at least one recess 24. The recess 24 can be cylindrical; however, it can be any shape, such as rectangular, oval, starred or triangular. The recess 24 can serve to enable the acetabular shell 12 to fixedly engage the bearing liner 14 in a desired position, and to provide axial alignment, as will be discussed in greater detail herein. The recess 24 can be formed on the interior surface 20 via any appropriate technique, such as casting or machining. A protrusion 25 from the exterior surface 22 can optionally be formed on the acetabular shell 12, as shown in phantom. The protrusion 25 can further assist in coupling the acetabular prosthesis assembly 10 to the pelvis 16 as will be discussed in greater detail herein; however, the protrusion 25 is not necessary. Generally, the recess 24 can be formed at an apex, dome or pole 26 of the acetabular shell 12; although the recess 24 can be positioned in any appropriate location.

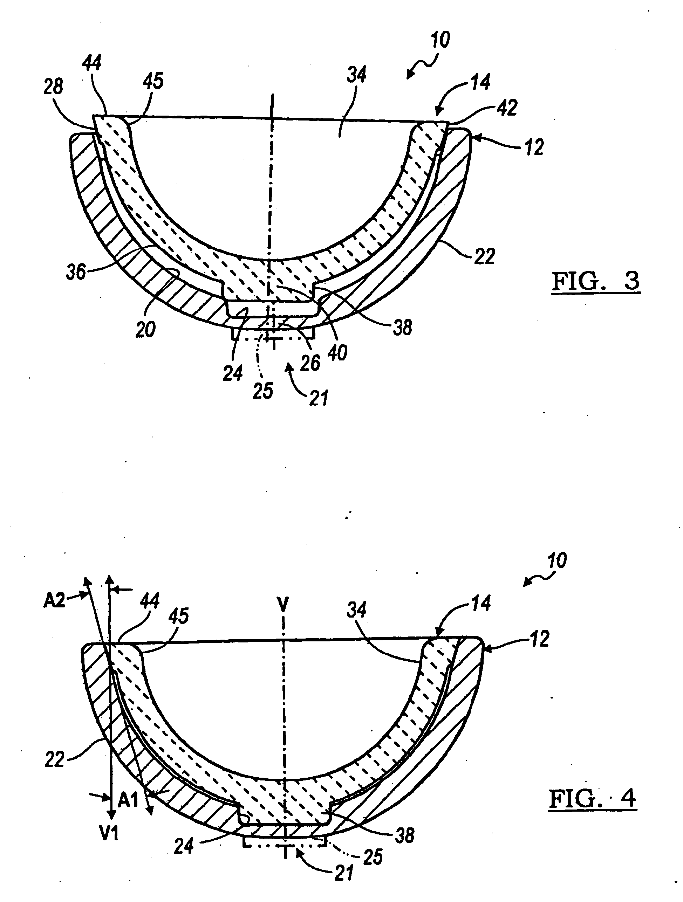

The interior surface 20 also includes a taper 28 formed on the interior surface 20 near a face or rim 30 of the acetabular shell 12. It will be understood, however, that although the taper 28 can be described herein as being formed near the rim 30, the taper 28 can be formed at any desired location, such as near a radial midpoint on the acetabular shell 12. The taper 28 can define any appropriate angle such as a locking taper. The taper 28 can be formed at a about 1° to about 25° angle A1 with respect to a vertical axis V1 (Fig. 4). The taper 28 can extend for a distance D1 along the rim 30 in either or both directions. Generally, the distance D1 is approximately 1 mm to approximately 20 mm. The taper 28 can be configured to frictionally engage the bearing liner 14 as will be discussed in greater detail herein.

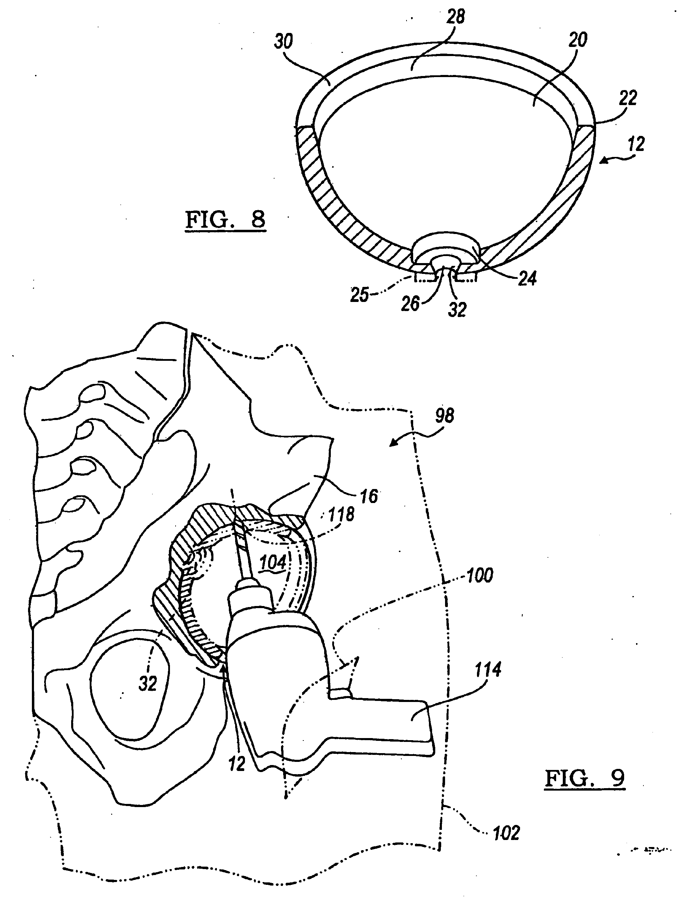

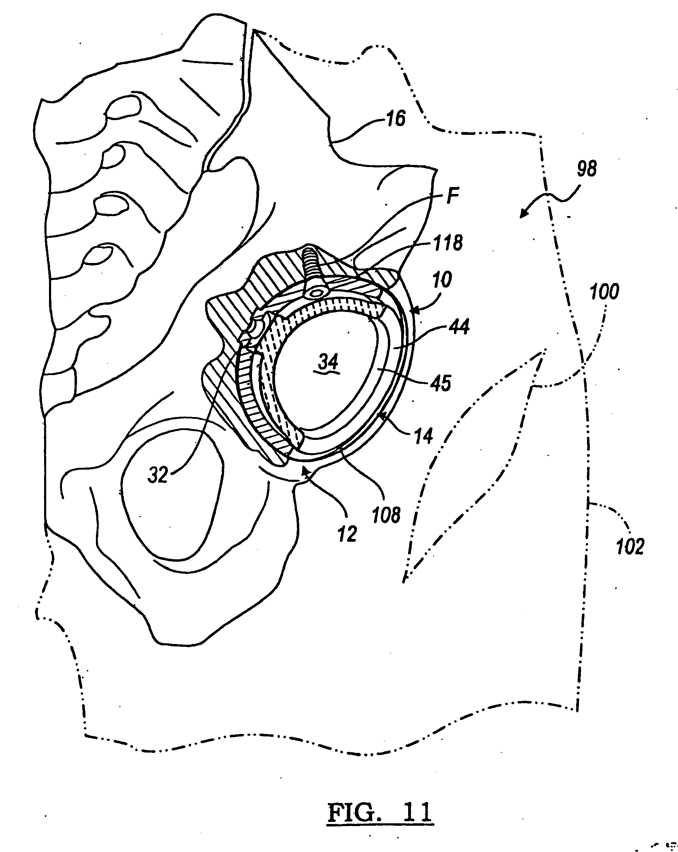

The exterior surface 22 of the acetabular shell 12 can secure the acetabular shell 12 to the portion of the anatomy, such as the pelvis 16. The exterior surface 22 can be coated with a bio-compatible material, such as plasma deposited porous coats, hydroxyapatite, calcium phosphate, porous metal matrix, or combinations thereof, to promote tissue growth between the acetabular shell 12 and the pelvis 16. The acetabular shell 12 can define at least one opening 32, as shown in Fig. 8. Alternatively, the acetabular shell 12 could be devoid of openings 32. The at least one opening 32, if employed, can be adapted to receive a bio-compatible fastener F, to couple the acetabular shell 12 to the pelvis 16, as will also be described herein (Fig. 11). The bio-compatible fastener F can be comprised of any suitable bio-compatible material, such as such as titanium, titanium alloy, stainless steel, cobalt-chromium-molybedenum alloy.

The interior surface 20 of the acetabular shell 12 can be configured to slideably engage the bearing liner 14. The bearing liner 14 is generally hemispherical, but could be any appropriate size for the desired application. The bearing liner 14 can be composed of a bio-compatible material, such as titanium, titanium alloy, stainless steel, cobalt-chromium-molybedenum alloy, ceramics, diamond compact, polycrystalline diamond compact, ultra high molecular weight polyethylene or combinations thereof. The bearing liner 14 can be generally concave and sized to mate with the femoral head prosthesis 120. The bearing liner 14 includes a first or interior surface 34. The interior surface 34 of the bearing liner 14 can be generally concave and smooth. The interior surface 34 can be configured to mate with the femoral head prosthesis 120 or other selected prostheses. The bearing liner 14 also includes a second or exterior surface 36.

The exterior surface 36 of the bearing liner 14 can secure the bearing liner 14 to the acetabular shell 12. The exterior surface 36 can include at least one projection 38. The projection 38 can be cylindrical; however, it can be any shape, such as rectangular, oval, starred or triangular, so long as the projection 38 can interact and/or mate with the recess 24 of the acetabular shell 12. The shape of the projection 38 can further provide rotational stability depending upon the shape employed, such as starred, oval, triangular or rectangular. The projection 38 can serve to axially align the bearing liner 14 within the acetabular shell 12 to allow the taper 28 of the acetabular shell 12 and a taper 42 of the bearing liner 14 to mate and lock the bearing liner 14 within the acetabular shell 12, as will be discussed in greater detail below. The projection 38 can be formed on the exterior surface 36 via any appropriate technique, such as casting or machining. Generally, the projection 38 can be formed at an apex, dome or pole 40 of the bearing liner 14. It will be noted that the engagement of the projection 38 with the recess 24 can provide a male to female connection between the bearing liner 14 and the acetabular shell 12. It will be understood, however, that the projection 38 could be formed on the acetabular shell 12 and the recess 24 could be formed on the bearing liner 14 to provide a female to male connection between the bearing liner 14 and the acetabular shell 12.

The exterior surface 36 of the bearing liner 14 can also include a taper 42 formed on the exterior surface 36 near a rim 44 of the bearing liner 14. The rim 44 can also include a radius or a chamfer 45 to enable smooth contact with the femoral head prosthesis 120. It will be understood, however, that although the taper 42 can be described herein as being formed near the rim 44, the taper 42 can be formed at any desired location, such as near a radial midpoint on the bearing liner 14. The taper 42 can define any appropriate angle such as a locking taper formed at about 1° to about 25° angle A2 with respect to vertical access V. The taper 42 may extend for a distance D2 along the rim 44. Generally, the distance D2 is approximately 1 mm to approximately 20 mm. The taper 42 can be configured to frictionally engage the taper 28 on the rim 30 of the acetabular shell 12.

In order to secure the bearing liner 14 within the acetabular shell 12, the bearing liner 14 can be placed into the acetabular shell 12. If the operator fails to place the projection 38 within the recess 24, the bearing liner 14 cannot seat within the acetabular shell 12, as shown in Fig. 3. The failure of the bearing liner 14 to seat within the acetabular shell 12 due to the failure of the projection 38 to mate with the recess 24 provides an obvious visual or tactile clue to the operator that the position of the bearing liner 14 is incorrect and the tapers 28, 42 are not locked. In effect, the projection 38 and recess 24 serve to provide a low cost error proofing feature.

Once the operator has the projection 38 engaging or interacting with the recess 24, the taper 42 on the bearing liner 14 can engage the taper 28 of the acetabular shell 12, as illustrated in Fig. 4. In general, the ability of the downward motion of the projection 38 into the recess 24 begins the engagement or interaction of the taper 28 on the acetabular shell 12 with the taper 42 of the bearing liner 14, such that once the projection 38 can be fully retained in the recess 24, the taper 28 on the acetabular shell 12 can be in complete frictional or locking engagement with the taper 42 of the bearing liner 14. Thus, the interaction of the projection 38 with the recess 24 can allow the bearing liner 14 to lock with the acetabular shell 12. In addition, this can ensure complete axial alignment between the acetabular shell 12 and bearing liner 14. It will be understood that locking can refer to an anatomically acceptable engagement between the bearing liner 14 and the acetabular shell 12. The lock between the tapers 28, 42 can be overcome, if selected, with an appropriate force or instrument, but generally is held during natural articulation once implanted.

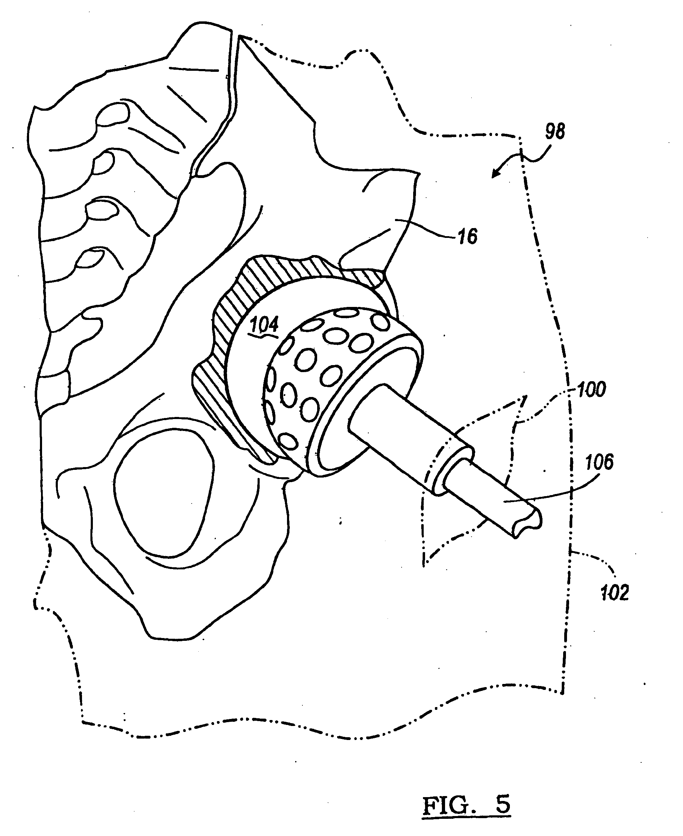

With reference now to Fig. 5, in order to secure the acetabular prosthesis assembly 10 to the anatomy 98, an incision 100 can be made into a selected portion of the skin 102 of a patient to provide access to the selected portion of the anatomy 98, in this case, the acetabulum 104 in the pelvis 16. The incision 100 can be any appropriate size, such as 1 cm to 20 cm and can include 1 cm to 10 cm. Then, the anatomy 98 can be reamed with a reamer 106 to provide a smooth interface for the acetabular shell 12. Next, the acetabular shell 12 can be secured to the acetabulum 104 in various distinct ways.

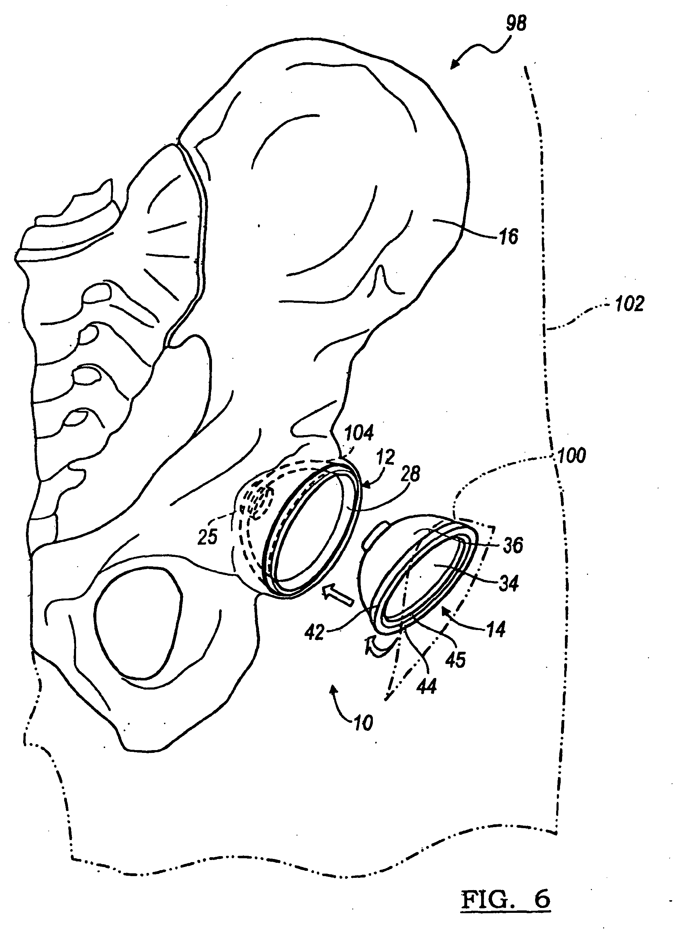

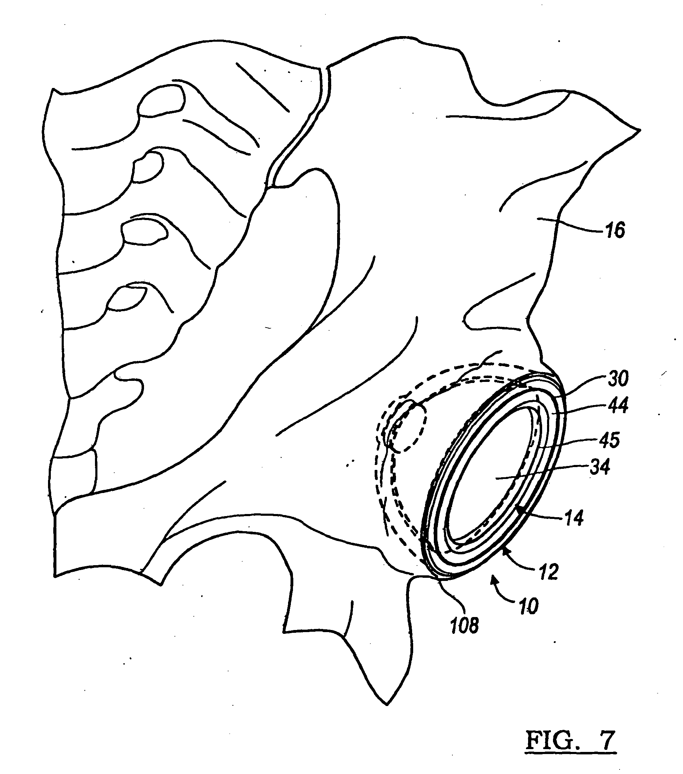

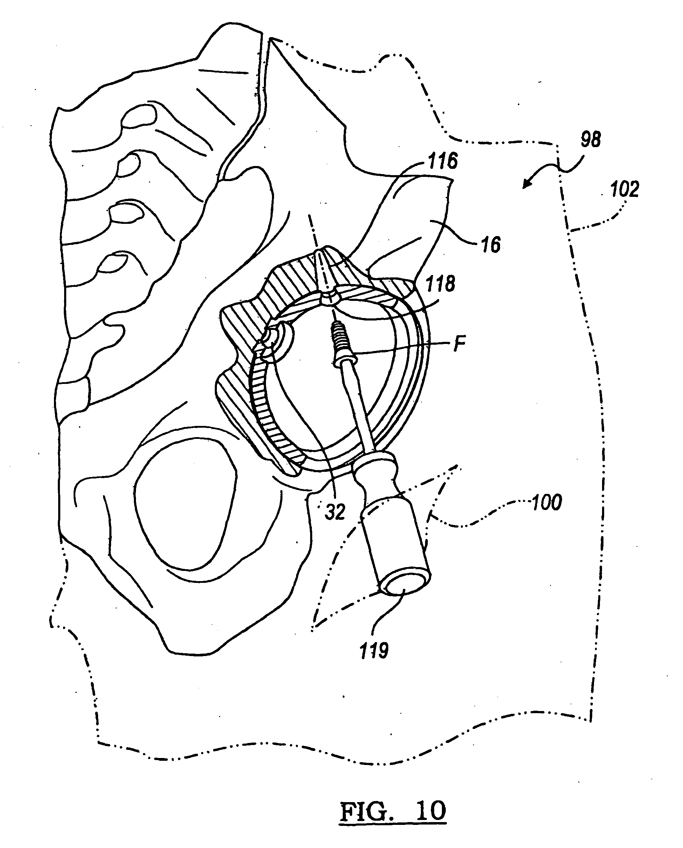

First, the acetabular shell 12 can be assembled and press-fitted into the acetabulum 104, as shown in Fig. 6. As illustrated in Fig. 7, the acetabular prosthesis assembly 10 once assembled can be generally flush with a surface 108 of the pelvis 16. Alternatively, as shown in Fig. 9, a drill 114 can be used to form at least one hole 116 in the acetabulum 104. It should be understood that the drill 114 can form the at least one hole 116 either before or after the acetabular shell 12 is positioned within the acetabulum, as shown in phantom. Then, as shown in Fig. 10, the bio-compatible fastener F can be inserted through at least one opening 118 formed in the acetabular shell 12. An operator can use a screwdriver 119 to screw the bio-compatible fastener F into the at least one hole 116 in the acetabulum 104 to secure the acetabular shell 12 to the acetabulum 104. It will be understood, however, that the at least one opening 118 in the acetabular shell 12 could be positioned at an alternative location and the hole 116 in the acetabulum 104 could also be positioned at an alternative location for receipt of the bio-compatible fastener F therethrough. In addition, the bio-compatible fastener F can also be screwed in with the screwdriver 119 either before or after the acetabular shell 12 is positioned in the acetabulum 104. The bearing liner 14 can then be inserted into the acetabular shell 12 to form the acetabular prosthesis assembly 10 as shown in Fig. 11.

Once the acetabular shell 12 is secured to the acetabulum 104, the bearing liner 14 can be secured to the acetabular shell 12. In order to secure the bearing liner 14 to acetabular shell 12, the operator may insert the bearing liner 14 through the incision 100. Next, the bearing liner 14 can be aligned within the acetabular shell 12. In order to align the bearing liner 14 within the acetabular shell 12, the operator may insert the bearing liner 14 into the acetabular shell 12 until the projection 38 fits within the recess 24. If the projection 38 is not aligned properly with the recess 24 of the acetabular shell 12, the bearing liner 14 may not sit within the acetabular shell 12, leaving a visual and/or tactile indicator to the operator that the bearing liner 14 is not properly aligned. Thus, the bearing liner 14 and acetabular shell 12 provide an obvious error-proofing feature ideal for minimally invasive procedures. If, however, the projection 38 of the bearing liner 14 engages the recess 24 of the acetabular shell 12, the taper 28 on the acetabular shell 12 can lock with the taper 42 of the bearing liner 14. Once the taper 28 on the acetabular shell 12 is locked with the taper 42 of the bearing liner 14, the acetabular prosthesis assembly 10 is fully assembled.

After the acetabular prosthesis assembly 10 is assembled within the acetabulum 104, the femoral prosthesis 18 can be coupled or positioned near to the bearing liner 14 of the acetabular prosthesis assembly 10, as shown in Fig. 12. The femoral prosthesis 18 can include an implant such as the femoral head prosthesis 120 sized for articulation within the bearing liner 14. Generally the size of the femoral head prosthesis 120 can be such that the femoral head prosthesis 120 resides entirely within the bearing liner 14, such that the bearing liner 14 forms an articulated bearing surface for the femoral head prosthesis 120. The femoral head prosthesis 120 can be formed out of any appropriate bio-compatible material, such as titanium, titanium alloy, stainless steel, cobalt-chromium-molybedenum alloy, ceramics, diamond compact, polycrystalline diamond compact, or combinations thereof.

The exterior surface 22 of the acetabular prosthesis assembly 10 can be coated with materials such as plasma-deposited porous coats, hydroxyapatite, calcium phosphate or the like to facilitate increased bone and tissue growth. Additionally, the use of a frictional taper lock between the bearing liner 14 and the acetabular shell 12 provides an internal locking mechanism which reduces the need for separate fasteners to engage the bearing liner 14 with the acetabular shell 12.

The description of these teachings is merely exemplary in nature and, thus, variations that do not depart from the gist of the teachings are intended to be within the scope of the teachings. Such variations are not to be regarded as a departure from the scope of the invention if encompassed by the claims.

## Claims

1. A prosthesis (10) for replacing a portion of the anatomy, comprising:
a first body (12) having a generally hemispherical first inner surface (20) comprising and extending between a first region and a second region and defining a first alignment portion (21) near the first region and a first taper (28) near the second region; and
a second body (14) having a generally hemispherical first exterior surface (36) comprising and extending between a third region and a fourth region and defining a second alignment portion (38) near the third region and a second taper (42) near the fourth region;
wherein the first body is adapted to receive the second body such that the second body is substantially disposed within the first body;
wherein the first alignment portion is adapted to interact with the second alignment portion and the first taper is adapted to interact with the second taper when the second body is received by the first body; and
wherein the first taper and the second taper interact when the first alignment portion and the second alignment portion interact.

2. The prosthesis of Claim 1, wherein the first region and the second region are a distance apart.

3. The prosthesis of Claim 1, wherein the first body defines a cavity, the first body further comprising:
a second exterior surface (22) configured to couple the first body to the anatomy,
wherein the first alignment portion includes at least one recess (24).

4. The prosthesis of Claim 3, wherein the second body defines a cavity, the second body further comprising:
a second inner surface (34) configured to engage a second portion of the anatomy,
wherein the second alignment portion includes at least one projection.

5. The prosthesis of Claim 4, wherein the at least one projection and the at least one recess are cylindrical.

6. The prosthesis of Claim 5, wherein the at least one projection is formed at a dome of the second body and the at least one recess is formed at a dome of the first body to prevent the first taper from interacting with the second taper until the at least one projection and at least one recess interact.

7. The prosthesis of Claim 6, wherein the at least one projection and at least one recess axially align the second body within the first body.

8. The prosthesis of Claim 4, wherein the first taper is formed near a rim (30) of the first body on the first surface and the second taper is formed near a rim (44) of the second body on the first surface.

9. The prosthesis of Claim 1, wherein the first body is an acetabular shell (12) and is composed of at least one material comprising: titanium, titanium alloy, stainless steel, cobalt-chromium-molybedenum alloy or combinations thereof.

10. The prosthesis of Claim 1, wherein the second body is a bearing liner (14) and is composed of at least one bio-compatible material comprising: metal, metal alloys, ceramic, ceramic diamond compact, polycrystalline diamond compact, ultra high molecular weight polyethylene or combinations thereof.

11. The prosthesis of Claim 1, wherein the second body is adapted to be coupled to a natural or prosthetic femoral head (120).

12. The prosthesis of Claim 1, wherein the interaction of the first taper and second taper form a taper lock.

13. The prosthesis of Claim 1, wherein the first alignment portion and first taper are formed on an inner surface of an acetabular shell.

14. The prosthesis of Claim 13, wherein the first alignment portion is formed at a dome of an acetabular shell and first taper is formed at a rim of the acetabular shell and the first alignment portion includes at least one recess.

15. The prosthesis of Claim 1, wherein the second alignment portion and second taper are formed on an exterior surface of an acetabular liner.

16. The prosthesis of Claim 1, wherein the second alignment portion is formed at a dome of the second body and second taper is formed at a rim of the second body and the second alignment portion includes at least one projection.

17. The prosthesis of Claim 1, wherein the first body is composed of a material selected from the group comprising: metal, metal alloy, ceramic, polymers.

18. The prosthesis of Claim 1, wherein the second body is composed of a bio-compatible material selected from the group comprising: metal, metal alloy, ceramic, polymers.

19. The prosthesis of Claim 1, wherein the first body further includes at least one opening (118) that receives a fastener for securing the prosthesis to a body part.

## Patentansprüche

1. Prothese (10) zum Ersetzen eines Teils der Anatomie, umfassend:
einen ersten Körper (12), der eine im Allgemeinen halbkugelförmige erste Innenfläche (20) aufweist, die einen ersten Bereich und einen zweiten Bereich umfasst und sie sich zwischen diesen erstreckt, und der einen ersten Ausrichtungsteil (21) nahe dem ersten Bereich und einen ersten konischen Ansatz (28) nahe dem zweiten Bereich definiert; und
einen zweiten Körper (14), der eine im Allgemeinen halbkugelförmige erste Außenfläche (36) aufweist, die einen dritten Bereich und einen vierten Bereich umfasst und sie sich zwischen diesen erstreckt, und der einen zweiten Ausrichtungsteil (38) nahe dem dritten Bereich und einen zweiten konischen Ansatz (42) nahe dem vierten Bereich definiert;
wobei der erste Köper den zweiten Körper derart aufnimmt, dass sich der zweite Körper im Wesentlichen im ersten Körper befindet;
wobei der erste Ausrichtungsteil mit dem zweiten Ausrichtungsteil interagiert und
der erste konische Ansatz mit dem zweiten konischen Ansatz interagiert, wenn der zweite Körper vom ersten Körper aufgenommen wird; und
wobei der erste konische Ansatz und der zweite konische Ansatz interagieren, wenn der erste Ausrichtungsteil und der zweite Ausrichtungsteil interagieren.

2. Prothese nach Anspruch 1, wobei der erste Bereich und der zweite Bereich in einem Abstand zueinander sind.

3. Prothese nach Anspruch 1, wobei der erste Körper einen Hohlraum definiert, wobei der erste Körper zudem umfasst:
eine zweite Außenfläche (22), die so konfiguriert ist, dass der erste Körper an der Anatomie befestigt ist,
wobei der erste Ausrichtungsteil mindestens eine Vertiefung (24) aufweist.

4. Prothese nach Anspruch 3, wobei der zweite Körper einen Hohlraum definiert, wobei der zweite Körper zudem umfasst:
eine zweite Innenfläche (34), die so konfiguriert ist, dass sie einen zweiten Teil der Anatomie greift,
wobei der zweite Ausrichtungsteil mindestens eine Erhebung aufweist.

5. Prothese nach Anspruch 4, wobei die mindestens eine Erhebung und die mindestens eine Vertiefung zylindrisch sind.

6. Prothese nach Anspruch 5, wobei die mindestens eine Erhebung an einer Kuppel des zweiten Körpers gebildet ist, und die mindestens eine Vertiefung an einer Kuppel des ersten Körpers gebildet ist, so dass verhindert wird, dass der erste konische Ansatz mit dem zweiten konischen Ansatz interagiert, bis die mindestens eine Erhebung und die mindestens eine Vertiefung interagieren.

7. Prothese nach Anspruch 6, wobei die mindestens eine Erhebung und die mindestens eine Vertiefung den zweiten Körper im ersten Körper axial ausrichten.

8. Prothese nach Anspruch 4, wobei der erste konische Ansatz nahe einer Kante (30) des ersten Körpers auf der ersten Oberfläche gebildet ist, und der zweite konische Ansatz nahe einer Kante (44) des zweiten Körpers auf der ersten Oberfläche gebildet ist.

9. Prothese nach Anspruch 1, wobei der erste Körper eine Hüftgelenksschale (12) ist und sie aus mindestens einem Material besteht, umfassend: Titan, Titanlegierung, Edelstahl, Kobalt-Chrom-Molybdän-Legierung oder Kombinationen davon.

10. Prothese nach Anspruch 1, wobei der zweite Körper ein Lagerfutter (14) ist und sie aus mindestens einem biologisch kompatiblen Material besteht, umfassend:
Metall, Metalllegierungen, Keramik, Keramik-Diamant-Pressling, polykristallinen Diamant-Pressling, Polyethylen mit ultrahohem Molekulargewicht oder Kombinationen davon.

11. Prothese nach Anspruch 1, wobei der zweite Körper an einen natürlichen oder prothetischen Femurkopf (120) gekoppelt ist.

12. Prothese nach Anspruch 1, wobei die Wechselwirkung des ersten konischen Ansatzes und des zweiten konischen Ansatzes eine Taperlock-Spannbuchse bildet.

13. Prothese nach Anspruch 1, wobei der erste Ausrichtungsteil und der erste konische Ansatz auf einer Innenfläche einer Hüftgelenksschale gebildet sind.

14. Prothese nach Anspruch 13, wobei der erste Ausrichtungsteil an einer Kuppel einer Hüftgelenksschale gebildet ist, und ein erster konischer Ansatz an einer Kante der Hüftgelenksschale gebildet ist, und der erste Ausrichtungsteil mindestens eine Vertiefung aufweist.

15. Prothese nach Anspruch 1, wobei der zweite Ausrichtungsteil und der zweite konische Ansatz auf einer Außenfläche eines Hüftgelenksfutters gebildet sind.

16. Prothese nach Anspruch 1, wobei der zweite Ausrichtungsteil an einer Kuppel des zweiten Körpers gebildet ist, und der zweite konische Ansatz an einer Kante des zweiten Körpers gebildet ist, und der zweite Ausrichtungsteil mindestens eine Erhebung aufweist.

17. Prothese nach Anspruch 1, wobei der erste Körper aus einem Material besteht, ausgewählt aus der Gruppe, die Metall, Metalllegierung, Keramik, Polymere umfasst.

18. Prothese nach Anspruch 1, wobei der zweite Körper aus einem biologisch kompatiblen Material besteht, ausgewählt aus der Gruppe, die Metall, Metalllegierung, Keramik, Polymere umfasst.

19. Prothese nach Anspruch 1, wobei der erste Körper zudem mindestens eine Öffnung (118) umfasst, die eine Halterung zum Sichern der Prothese an einem Körperteil aufnimmt.

## Revendications

1. Prothèse (10) pour remplacer une partie de l'anatomie, comprenant :
- un premier élément (12) comportant une première surface intérieure généralement hémisphérique (20) comprenant et s'étendant entre une première région et une deuxième région et définissant une première partie d'alignement (21) proche de la première région et un premier amincissement conique (28) proche de la deuxième région ; et
- un deuxième élément (14) comportant une première surface extérieure généralement hémisphérique (36) comprenant et s'étendant entre une troisième région et une quatrième région et définissant une deuxième partie d'alignement (38) proche de la troisième région et un deuxième amincissement conique (42) proche de la quatrième région ;
- dans laquelle le premier élément est adapté à recevoir le deuxième élément de telle sorte que le deuxième élément soit disposé sensiblement dans le premier élément ;
- dans laquelle la première partie d'alignement est adaptée à interagir avec la deuxième partie d'alignement et le premier amincissement conique est adapté à interagir avec le deuxième amincissement conique lorsque le deuxième élément est reçu par le premier élément ; et
- dans laquelle le premier amincissement conique et le deuxième amincissement conique interagissent lorsque la première partie d'alignement et la deuxième partie d'alignement interagissent.

2. Prothèse selon la revendication 1, dans laquelle la première région et la deuxième région sont séparées par une certaine distance.

3. Prothèse selon la revendication 1, dans laquelle le premier élément définit une cavité, le premier élément comprenant en outre :
- une deuxième surface extérieure (22) configurée pour accoupler le premier élément à l'anatomie,
- dans laquelle la première partie d'alignement comporte au moins une cavité (24).

4. Prothèse selon la revendication 3, dans laquelle le deuxième élément définit une cavité, le deuxième élément comprenant en outre :
- une deuxième surface intérieure (34) configurée pour venir au contact d'une deuxième partie de l'anatomie,
- dans laquelle la deuxième partie d'alignement comporte au moins une saillie.

5. Prothèse selon la revendication 4, dans laquelle l'au moins une saillie et l'au moins une cavité sont cylindriques.

6. Prothèse selon la revendication 5, dans laquelle l'au moins une saillie est formée au niveau d'un dôme du deuxième élément et l'au moins une cavité est formée au niveau d'un dôme du premier élément pour empêcher le premier amincissement conique d'interagir avec le deuxième amincissement conique jusqu'à ce que l'au moins une saillie et l'au moins une cavité interagissent.

7. Prothèse selon la revendication 6, dans laquelle l'au moins une saillie et l'au moins une cavité alignent axialement le deuxième élément dans le premier élément.

8. Prothèse selon la revendication 4, dans laquelle le premier amincissement conique est formé près d'une couronne (30) du premier élément sur la première surface et le deuxième amincissement conique est formé près d'une couronne (44) du deuxième élément sur la première surface.

9. Prothèse selon la revendication 1, dans laquelle le premier élément est une enveloppe acétabulaire (12) et est composé d'au moins un matériau parmi : le titane, un alliage de titane, un acier inoxydable, un alliage de cobalt/chrome/molybdène ou des combinaisons de ceux-ci.

10. Prothèse selon la revendication 1, dans laquelle le deuxième élément est un manchon d'articulation (14) et est composé d'au moins un matériau biologiquement compatible parmi : un métal, des alliages de métaux, une céramique, un diamant céramique compacté, un diamant polycristallin compacté, un polyéthylène de masse moléculaire ultra-haute ou des combinaisons de ceux-ci.

11. Prothèse selon la revendication 1, dans laquelle le deuxième élément est adapté à être accouplé à une tête fémorale naturelle ou prothétique (120).

12. Prothèse selon la revendication 1, dans laquelle l'interaction du premier amincissement conique et du deuxième amincissement conique forme un verrouillage conique.

13. Prothèse selon la revendication 1, dans laquelle la première partie d'alignement et le premier amincissement conique sont formés sur une surface intérieure d'une enveloppe acétabulaire.

14. Prothèse selon la revendication 13, dans laquelle la première partie d'alignement est formée au niveau d'un dôme d'une enveloppe acétabulaire, le premier amincissement conique est formé au niveau d'une couronne de l'enveloppe acétabulaire et la première partie d'alignement comporte au moins une cavité.

15. Prothèse selon la revendication 1, dans laquelle la deuxième partie d'alignement et le deuxième amincissement conique sont formés sur une surface extérieure d'un manchon acétabulaire.

16. Prothèse selon la revendication 1, dans laquelle la deuxième partie d'alignement est formée au niveau d'un dôme du deuxième élément, le deuxième amincissement conique est formé au niveau d'une couronne du deuxième élément et la deuxième partie d'alignement comporte au moins une saillie.

17. Prothèse selon la revendication 1, dans laquelle le premier élément est composé d'un matériau sélectionné parmi le groupe constitué par : un métal, un alliage métallique, une céramique et des polymères.

18. Prothèse selon la revendication 1, dans laquelle le deuxième élément est composé d'un matériau biologiquement compatible sélectionné parmi le groupe constitué par : un métal, un alliage métallique, une céramique et des polymères.

19. Prothèse selon la revendication 1, dans laquelle le premier élément comporte en outre au moins une ouverture (118) recevant une attache pour fixer la prothèse sur une partie du corps.
